(19) Europäisches Patentamt
European Patent Office
Office européen des brevets

(11) Publication number : 0 511 168 A1

(12) **EUROPEAN PATENT APPLICATION**

(21) Application number : 92810290.4

(22) Date of filing : 22.04.92

(51) Int. Cl.$^5$ : C07D 209/32, A61K 31/40

(30) Priority : 26.04.91 GB 9108965

(43) Date of publication of application : 28.10.92 Bulletin 92/44

(84) Designated Contracting States : AT BE CH DE DK ES FR GB GR IT LI LU NL PT SE

(71) Applicant : SANDOZ LTD.
Lichtstrasse 35
CH-4002 Basel (CH)
(84) BE CH DK ES FR GB GR IT LI LU NL PT SE

(71) Applicant : SANDOZ-PATENT-GMBH
Humboldtstrasse 3
W-7850 Lörrach (DE)
(84) DE

(71) Applicant : SANDOZ ERFINDUNGEN VERWALTUNGSGESELLSCHAFT M.B.H.
Brunner Strasse 59
A-1235 Vienna (AT)
(84) AT

(72) Inventor : Achini, Roland
Vogesenstrasse 33
Ch-4106 Therwil (CH)
Inventor : Fünfschilling, Peter
Ochsengasse 62
CH-4123 Allschwil (CH)

(54) **Indoline hydrochloride salts and process for their preparation.**

(57) The invention relates to 3,3-dimethyl-6-ethoxy-indoline hydrochloride, preferably in the crystalline A modification, and to a process for its production. 3,3-dimethyl-6-ethoxy-indoline hydrochloride possesses analgesic activity.

EP 0 511 168 A1

The present invention relates to indoline hydrochloride salts having pharmaceutical utility, a process for their production, pharmaceutical compositions comprising them and their use as pharmaceuticals.

USP 4,622,336 discloses the compound 3,3-dimethyl-6-ethoxy-indoline [hereinafter compound I]. It discloses the preparation of compound I in the form of its hydrobromide salt and also its action in the arthritis test and the Randall-Selitto test in rats. Compound I in the form of its methane sulfonate has also been disclosed in Life Sciences, 43, 905-912 (1988).

The hydrobromide and the methane sulfonate salts of compound I are both prepared from compound I in free base form. However compound I in free base form is less stable than the corresponding hydrobromide or the methane sulfonate, e.g. it is sensitive to oxidation, and this impairs the production of the salt form in large quantities.

We have now found that the hydrochloride salt of compound I possesses especially advantageous properties, in particular relating to its chemical production.

More particularly, it has been found that the hydrochloride salt of compound I may exist in the form of various crystalline modifications. At least 6 polymorphic or pseudopolymorphic modifications have been identified: they are defined as modifications A, B, C, D, E and F. The present invention is to be understood as embracing the various crystalline modifications of the hydrochloride of compound I in substantially pure form, as well as in hydrated form or mixtures thereof.

The modifications A and C are anhydrous forms. The modifications E and F are monohydrates.

The modification A is the form which is thermodynamically stable at room temperature. It is also resistant to mechanical stress. The modification C is the form which is thermodynamically stable at a higher temperature, e.g. 120 to 150°C (enantiotropy between A and C). Form C may be obtained by heating form A up to 120-130°C. The modification C may be converted to modification A by mechanical stress, e.g. grinding. A 1:1 mixture of forms A and C may be converted into pure form A by maintaining the mixture for 1 day at 0° or 25°C in the absence of any stress. m.p. of form C = 146°C.

The modification E is obtained by incubating form A at a relative humidity > 65%. It may be converted into the pure form A under mild drying conditions (in vacuo, 1 day at 50°C). m.p. = 82°C.

The modification F may be obtained by crystallisation of form A in aqueous solution.

To assist identification of the modifications A and C, X-ray powder diffraction data are provided in the following Tables I and II. Attached Figures I and II provide a representation of the X-ray powder diffraction diagram from which the data in Tables I and II are obtained. The diffraction diagram was obtained employing a XDS 2000 powder diffractometer (Scintag Inc.), using CuKα-radiation, λ = 1.54060 Å.

**TABLE I: Modification A**

| Peak No. | d-value in Å | $I/I_1$ |
|---|---|---|
| 5. | 5.885 | 100 |
| 11. | 3.633 | 46 |
| 3. | 7.237 | 33 |
| 4. | 5.954 | 32 |
| 2. | 7.996 | 24 |
| 13. | 3.534 | 21 |
| 8. | 5.062 | 19 |
| 10. | 4.124 | 18 |
| 9. | 4.541 | 14 |
| 21. | 2.744 | 13 |
| 12. | 3.562 | 9 |

**TABLE II: Modification C**

| Peak No. | d-value in Å | $I/I_1$ |
|---|---|---|
| 2. | 6.967 | 100 |
| 8. | 4.503 | 47 |
| 1. | 10.415 | 31 |
| 15. | 3.527 | 31 |
| 10. | 4.228 | 28 |
| 9. | 4.340 | 23 |
| 11. | 4.024 | 20 |
| 17. | 3.155 | 18 |
| 18. | 3.011 | 14 |
| 5. | 5.022 | 12 |
| 7. | 4.543 | 11 |
| 4. | 6.260 | 11 |
| 6. | 4.834 | 10 |

The modifications A and C are preferred. The hydrochloride of compound I in form A is particularly preferred.

The present invention also provides a process for the production of the hydrochloride of compound I, which process comprises etherifying a compound of formula II

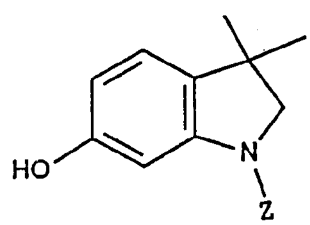

II

wherein Z is a protecting group, and then removing the protecting group Z in the presence of hydrochloric acid.

Examples of suitable protecting groups as Z in the compound of formula II include acyl groups, e.g. $C_2$romanfont250subfont12 kanoyl or benzoyl, preferably acetyl.

The first step of the production process is an etherification of the hydroxy group in position 6. It may be carried out in analogous manner to known alkylating method, e.g. using an ethylating agent, e.g. diethyl sulfate or diethyl carbonate. Diethyl sulfate is a preferred ethylating agent. The reaction may preferably be carried out at a an alkaline pH, e.g. a pH of from 10 to 13, conveniently at a temperature of from room temperature to 60°C. Advantageously an inert solvent or diluent such as e.g. hexane or heptane may be used.

The removal of the protecting group Z may be carried out in the presence of HCl in water. Preferably however the protecting group Z is removed under substantially water-free conditions.

According to a preferred embodiment of the invention a compound of formula III

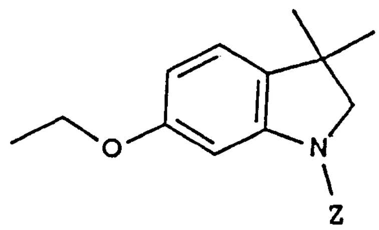

III

wherein Z is as defined above, is treated with gaseous HCl, preferably under substantially water-free conditions. The reaction may conveniently be carried out in a solvent, e.g. an alcohol such as methanol, ethanol, propanol or butanol, preferably methanol or propanol. The reaction may conveniently be performed at a temperature of from room temperature up to a temperature below the boiling temperature of the solvent used.

The reaction is preferably performed in the absence of any external water addition. The solvent is preferably used in anhydrous form. A small amount of water may be produced in the reaction mixture due to the reaction of gaseous HCl with the alcohol when the solvent used is an alcohol. However, this takes place in a limited extent and has no significant effect on the production of the final product.

The removal of the protecting group Z with gaseous HCl is caused by a transfer of the protecting group onto the solvent, e.g. alcohol. In the case where acetyl is used as a protecting group and methanol is employed as solvent, methyl acetate is formed.

The removal of Z in the presence of gaseous HCl facilitates the obtention of the hydrochloride via an intermediary immonium chloride salt, thus avoiding the intermediate formation of the compound I in free base form. As already mentioned; compound I in free base form is sensitive to oxidation. The substantially water-free conditions of the last step of the process of the invention result in the direct production of the hydrochloride in the crystalline form A with a high yield.

When Z is e.g. $C_2$*romanfont*250*subfont*128–8alkanoyl, the following intermediate immonium chloride compound of formula IIIa is formed:

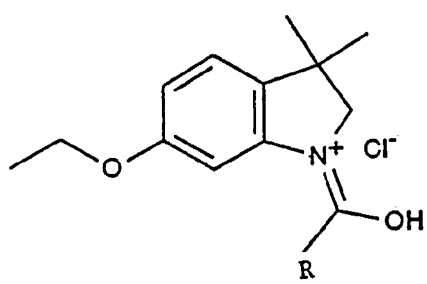

IIIa

wherein R is $C_1$*romanfont*250*subfont*128–7alkyl. It may be isolated in crystalline form

Compound of formula IIIa is new and forms also part of the invention. R is preferably $C_1$*romanfont*250*subfont*128–4alkyl, more preferably methyl.

In the process according to the invention, compound of formula IIIa may also be used directly without isolation in the next process step.

Compounds of formula II, used as starting material, may be prepared e.g. according to the following reaction scheme:

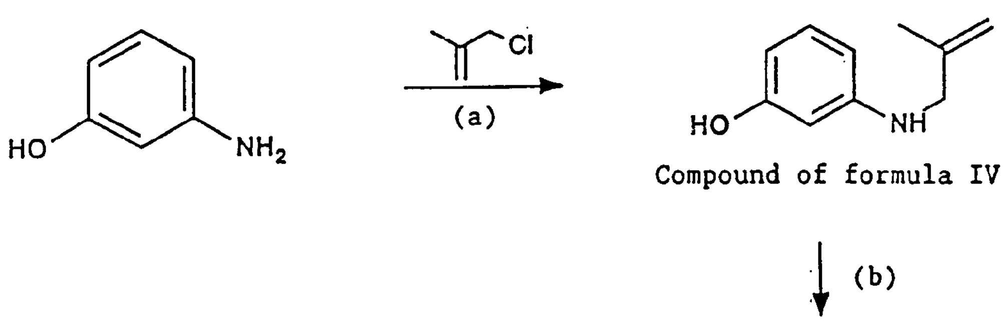


Compound of formula IV

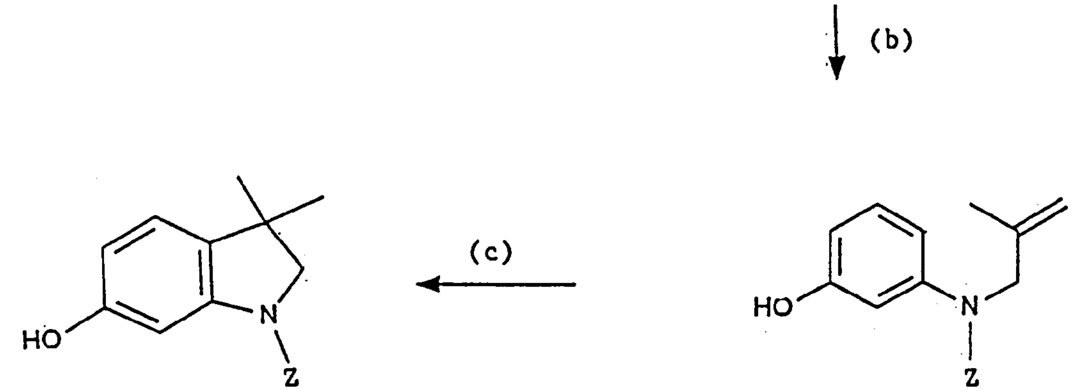


Compound of formula II

Process step a) may conveniently be carried out at a temperature of from 50 to 80°C. Preferably the pH of the reaction medium is adjusted to a slightly acid value. A suitable solvent includes e.g. toluene.

Process step b) is the protecting step, e.g. by acylation and may be carried out in analogy to known methods. If desired, process steps a) and b) may be performed as a one pot process without isolating the compounds of formula IV.

The cyclisation step c) may advantageously be carried out in the presence of aluminium chloride. The temperature of the reaction may advantageously be of from 85 to 130°C.

The hydrochloride salt of compound I has been found to possess analgesic activity comparable to that of the hydrobromide or methane sulfonate salt. For example in the Randall-Selitto paw pressure model in the hind paw of the rat an $ED_{50}$ of 79 mg/kg p.o. was determined for the hydrochloride salt (compare $ED_{50}$ of 78 mg/kg p.o. for the hydrobromide salt and of 79 mg/kg p.o. for the methane sulfonate). In the arthritis pain test in the rat [based on the method of A. W. Pircio et al. Eur. J. Pharmacol., 31, 207-215 (1975)] an $ED_{50}$ of 7.2 mg/kg p.o. was determined for the hydrochloride salt which is at a comparable level to the hydrobromide or the methane sulfonate.

The hydrochloride salt is useful for the same indications as known for the hydrobromide or methane sulfonate salt; e.g. in the treatment of pain. For this use the dosage will, of course, vary depending on e.g. the mode of administration, the condition to be treated (e.g. the aetiology of the pain to be treated) and the effect desired. However, in general, satisfactory results are obtained on administration at daily dosages of from about 100 to 600 mg. Suitable dosage forms, e.g. for oral administration, may contain from about 25 to 300 mg of salt.

In accordance with the foregoing the present invention also provides

i. The hydrochloride salt of 3;3-dimethyl-6-ethoxy-indoline for use as a pharmaceutical; e.g. for use as an analgesic;

ii. A method for treating or alleviating pain in a subject in need of such treatment, which method comprises administering to said subject an analgesically effective amount of the hydrochloride salt of 3,3-dimethyl-6-ethoxy-indoline;

iii. A pharmaceutical composition comprising the hydrochloride salt of 3,3-dimethyl-6-ethoxy-indoline together with a pharmaceutically acceptable diluent or carrier therefor.

The hydrochloride salt may be administered by any conventional route, in particular nasally, enterally, preferably orally, e.g. in the form of tablets or capsules, or parenterally e.g. in the form of injectable solutions or suspensions or in a suppository form.

In the case of oral dosage forms, e.g. containing up to 250 mg or 500 mg (expressed as base), the hydrochloride salt of the invention facilitates the obtention of formulations with an optimized weight.

The hydrochloride salt has been found to be well tolerated. Both the hydrochloride salt and the hydrobromide salt produce no ulcers in normally fed rat at a daily dose of 560 mg/kg/day p.o. for 5 days.

Preferably the hydrochloride salt of 3,3-dimethyl-6-ethoxy-indoline is used in the A form.

The following examples are illustrative of the process of the invention.

**EXAMPLE 1 : 3,3-Dimethyl-6-ethoxy-indoline hydrochloride**

i. 102.6 g (0.50 M) N-acetyl-3,3-dimethyl-6-hydroxy-indoline; 820 ml hexane and 375 ml water are heated to 45°C while stirring. By adding rapidly dropwise 50.1 ml 30% NaOH, the starting compound dissolves

and a pH of 12.5 is reached. Thereafter 138.8 g (0.9 M) diethyl sulfate are added dropwise within 15 minutes at 45° C. The reaction is slightly exothermic and the pH decreases to 12.2. The reaction mixture is further stirred for 4.5 hours at 45°C. The pH continuously decreases and each time it reaches the value of 11 a portion of 5 ml NaOH is added. In total 20 ml NaOH are added, thus maintaining the pH between 11 and 12. Excess of diethyl sulfate is destroyed by the addition of 62.5 ml 25% ammonia and stirring for 3 hours at 45°C.

The reaction mixture is heated to 55° C, the phases are separated, the organic phase is extracted with 100 ml water at 55°C and gradually cooled while stirring. Crystallisation starts at ca. 48°C. The mixture is stirred overnight at room temperature and then for 1 hour with ice cooling and filtrated. The crystals are rinsed twice with 25 ml hexane and dried for 2 hours at 40°C and 10 mm Hg, thus yielding N-acetyl-3,3-dimethyl-6-ethoxy-indoline. m.p. = 81-83°C.

ii. 166.6 g (0.5 M) of the compound obtained in step i. above are introduced in 200 ml methanol and heated to 55°C while stirring, thus giving a clear solution. 22.3 g (0.61 M) gaseous HCl are then introduced within 1 hour at 55-58°C. The intermediary immonium chloride salt precipitates but is not isolated. After further stirring for ca. 2 hours a clear solution is obtained. After a total of 3 hours stirring 3.2 g (0.088 M) gaseous HCl are further added, the mixture is stirred at 55°C overnight, and then clarified by filtration.

100 g Methanol/methyl acetate are distilled off under a mild vacuum. 230 ml Isopropyl acetate are added and 198 ml methanol/methyl acetate/isopropyl acetate are distilled off. 130 ml Isopropyl acetate are finally added at 50-55°C. The mixture is cooled to 25°C for 2 hours. At ca. 50°C crystallization of the product starts. The suspension is filtrated, the residue is washed 3 times with 25 ml isopropyl acetate and dried for ca. 15 hours at 50°C and under 10 mm Hg, thus yielding the title compound in form A. m.p. 113-114.8°C

The starting material may be prepared as follows:

a. 141.8 g (1.3 M) m-amino-phenol are suspended in 100 ml toluene and 100 ml water at 70°C giving a pH of 6.5. 90.5 g (1.00 M) methallyl chloride are added dropwise at 70-73°C within 70 minutes. After the addition of ca. 50% the pH has decreased to 4. When this value is reached, at the beginning simultaneously with the addition of methallyl chloride, 97 ml (0.95 M) 30% NaOH are added dropwise over 4.5 hours at such a rate that the pH is between 4 and 5 until the addition of ca. 85% of the NaOH amount has been achieved. The remaining 15% NaOH are added keeping the pH between 5-7. The phases are separated at 70°C. The organic phase is extracted 8 times with 100 ml water at 70°C to remove the excess of m-aminophenol and the combined aqueous phases are extracted with 100 ml toluene at 45°C. In addition to 3-N-methallylamino-phenol, the toluene phase comprises also a minor amount of 3-N-bis(methallyl)amino-phenol as a by-product.

b. 68.9 g (0.675 M) acetic anhydride are added dropwise at 40-50°C within 45 minutes to the combined toluene phase (556 g) obtained above and pre-heated at 40°C. At 45-50°C 100 ml water are then added and subsequently the pH is adjusted to 8.0 by dropwise addition of 70 ml 30% NaOH at 45-50°C within 10 minutes. The phases are separated, the toluene phase is washed twice with 100 ml water and the combined aqueous phases are extracted with 100 ml toluene. The combined toluene phases are evaporated, yielding a dark oil-like residue which can be used tel quel in the next step. It may also be purified by crystallisation in isopropyl ether. m.p. = 91-92°C

c. 269.5 g (2.02 M) aluminium chloride and 270 ml o-dichlorobenzene are heated to 85°C with stirring. A solution of 164 g of the residue obtained above in 140 ml o-dichlorobenzene are added dropwise within 80 minutes at a temperature of from 85 to 90°C. This addition is relatively exothermic. The resulting solution is heated for 3 hours at 110°C and for 1 hour at 130°C. From ca. 105°C HCl is continuously evolving.

The reaction mixture cooled to 90°C is poured on 750 g ice, which gives a temperature of from 78°C. The mixture is further heated to reflux for 1 hour and then allowed to cool to room temperature. The precipitate is filtered off, washed with 100 ml water and then with 200 ml methyl isobutyl alcohol and dried overnight in vacuo at 90°C. The residue contains also a small amount of the 7-hydroxy isomer which is removed by dissolution of the crude product in 1700 ml methyl isobutyl alcohol with stirring at reflux at 130°C and gradual cooling. Crystallisation of the N-acetyl-3,3-dimethyl-6-hydroxy-indoline starts at ca. 80°C. The mixture is allowed to cool to room temperature and then filtrated. The light beige residue is washed with 150 ml methyl isobutyl alcohol and dried overnight at 90°C. m.p. = 221-222.3°C

In process step ii. above the intermediary immonium salt can also be isolated. m.p. = 140° C with decomposition

## Claims

**1.** 3,3-dimethyl-6-ethoxy-indoline hydrochloride.

2. 3,3-dimethyl-6-ethoxy-indoline hydrochloride anhydrate, in the crystalline A modification stable at room temperature.

3. The compound according to claim 2 having a diffraction diagram as follows:

| Peak No. | d-value in Å | $I/I_1$ |
|---|---|---|
| 5. | 5.885 | 100 |
| 11. | 3.633 | 46 |
| 3. | 7.237 | 33 |
| 4. | 5.954 | 32 |
| 2. | 7.996 | 24 |
| 13. | 3.534 | 21 |
| 8. | 5.062 | 19 |
| 10. | 4.124 | 18 |
| 9. | 4.541 | 14 |
| 21. | 2.744 | 13 |
| 12. | 3.562 | 9 |

employing a XDS 2000 powder diffractometer (Scintag Inc.) using CuKα-radiation, λ = 1.54060 Å.

4. 3,3-dimethyl-6-ethoxy-indoline hydrochloride, in the crystalline C modification having a diffraction diagram as follows:

| Peak No. | d-value in Å | $I/I_1$ |
|---|---|---|
| 2. | 6.967 | 100 |
| 8. | 4.503 | 47 |
| 1. | 10.415 | 31 |
| 15. | 3.527 | 31 |
| 10. | 4.228 | 28 |
| 9. | 4.340 | 23 |
| 11. | 4.024 | 20 |
| 17. | 3.155 | 18 |
| 18. | 3.011 | 14 |
| 5. | 5.022 | 12 |
| 7. | 4.543 | 11 |
| 4. | 6.260 | 11 |
| 6. | 4.834 | 10 |

employing a XDS 2000 powder diffractometer (Scintag Inc.) using cuKα-radiation, λ= 1.54060 Å.

5. A process for producing the hydrochloride salt of 3,3-dimethyl-6-ethoxy-indoline which process comprises etherifying a compound of formula II

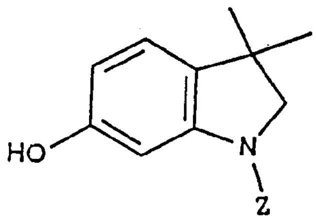

II

wherein Z is a protecting group and then removing the protecting group Z in the presence of hydrochloric acid.

**6.** A process according to claim 5 wherein a compound of formula III

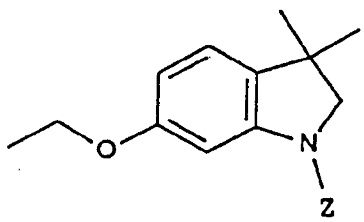

III

wherein
Z is a protecting group,
is treated with gaseous HCl.

**7.** 3,3-dimethyl-6-ethoxy-indoline hydrochloride, whenever prepared by a process according to claim 5 or 6.

**8.** Use of 3,3-dimethyl-6-ethoxy-indoline hydrochloride according to any one of claim 1, 2, 3 and 7 as a pharmaceutical.

**9.** A pharmaceutical composition comprising 3,3-dimethyl-6-ethoxyindoline hydrochloride according to any one of claims 1, 2, 3 and 7, together with a pharmaceutically acceptable diluent or carrier therefor.

**10.** A compound of formula IIIa

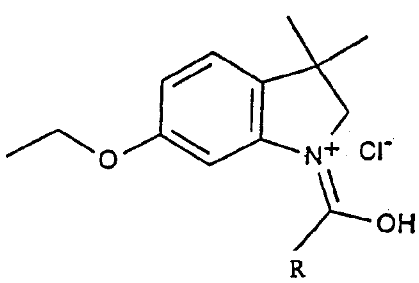

IIIa

wherein
R is $C_1$*romanfont*250*subfont*128–7alkyl.

FIGURE I : MODIFICATION A

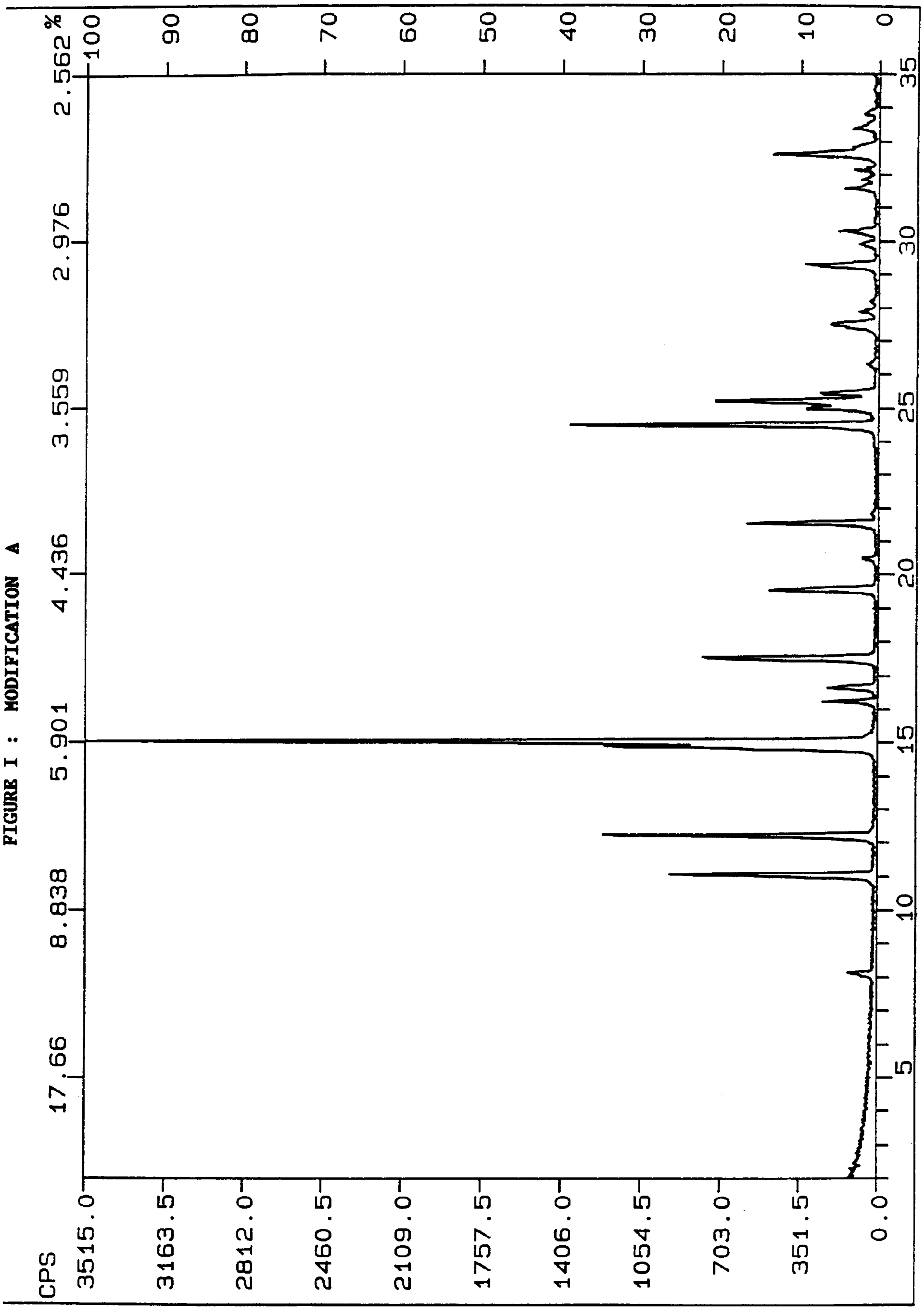

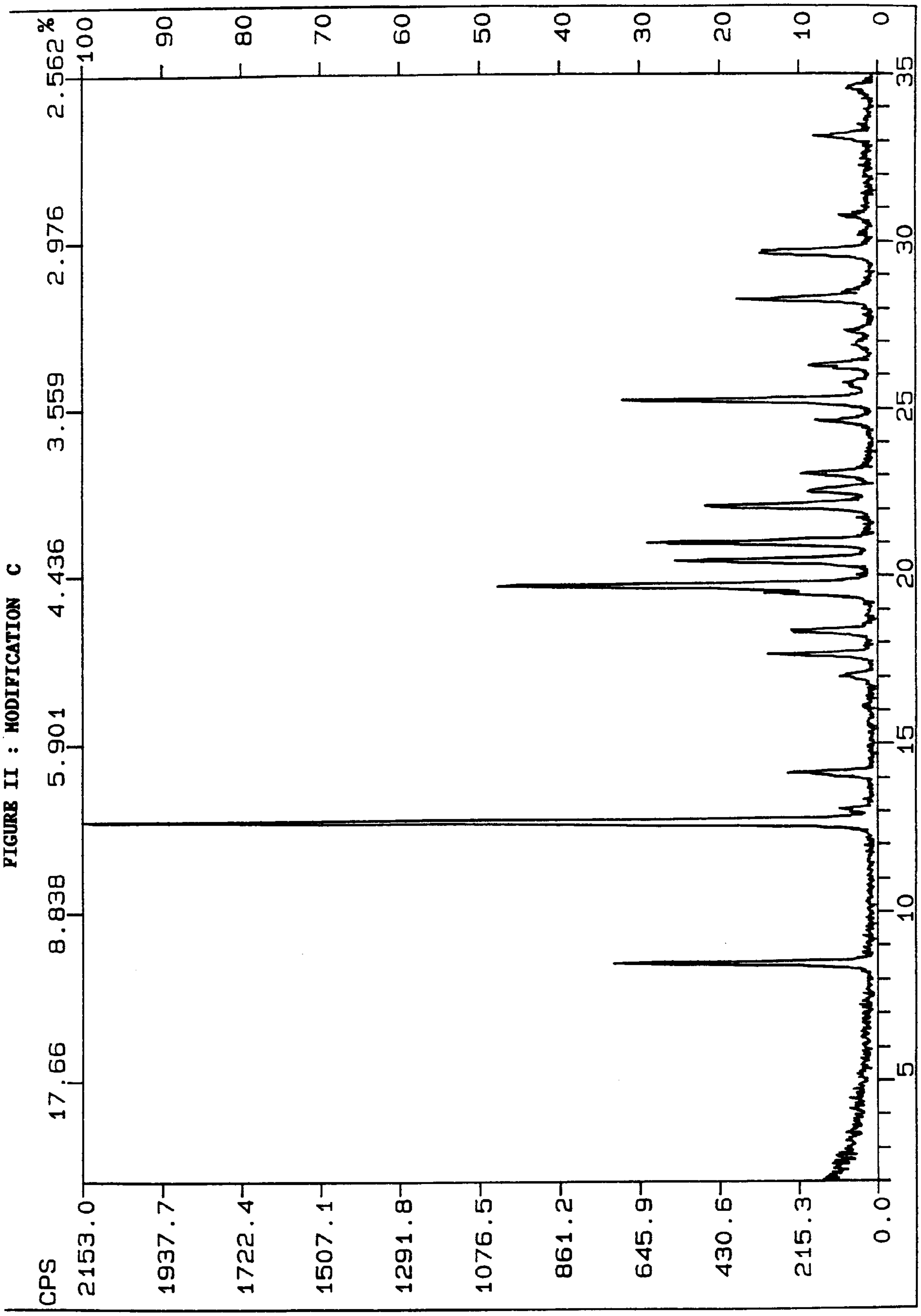


FIGURE II : MODIFICATION C

European Patent Office

# EUROPEAN SEARCH REPORT

Application Number

EP 92 81 0290

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 622 336 (ACHINI) 11 November 1986<br>formula (I), column 1 and Example 6, column 9<br>--- | 1-9 | C07D209/32<br>A61K31/40 |
| D,X | CHEMICAL ABSTRACTS, vol. 109, no. 19, 7 November 1988, Columbus, Ohio, US; abstract no. 163287G, R. FOOTE: 'FS 205-397: a new antipyretic analgesic with a paracetamol-like profile of activity'<br>page 51 ;column 1 ;<br>* abstract *<br>----- | 1-9 | |
| | | | TECHNICAL FIELDS SEARCHED (Int. Cl.5) |
| | | | C07D<br>A61K |

The present search report has been drawn up for all claims

| Place of search | Date of completion of the search | Examiner |
|---|---|---|
| MUNICH | 08 JULY 1992 | GETTINS M.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)